(19) Europäisches Patentamt
European Patent Office
Office européen des brevets

(11) **EP 4 194 053 B1**

(12) **EUROPEAN PATENT SPECIFICATION**

(45) Date of publication and mention
of the grant of the patent:
**08.10.2025 Bulletin 2025/41**

(21) Application number: **21855671.0**

(22) Date of filing: **09.08.2021**

(51) International Patent Classification (IPC):
***A61N 5/10*** (2006.01)

(52) Cooperative Patent Classification (CPC):
**A61N 5/1078; H05H 3/06; H05H 6/00;**
A61N 2005/109; A61N 2005/1094

(86) International application number:
**PCT/ES2021/070607**

(87) International publication number:
**WO 2022/034257 (17.02.2022 Gazette 2022/07)**

(54) **DEVICE FOR THE PRODUCTION, MODERATION AND CONFIGURATION OF NEUTRON BEAMS FOR NEUTRON CAPTURE THERAPY**

VORRICHTUNG ZUR ERZEUGUNG, MODERATION UND KONFIGURATION VON NEUTRONENSTRAHLEN FÜR EINE NEUTRONENEINFANGTHERAPIE

DISPOSITIF DE PRODUCTION, DE MODÉRATION ET DE MISE EN FORME DE FAISCEAUX DE NEUTRONS POUR LA THÉRAPIE PAR CAPTURE NEUTRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **09.08.2020 ES 202030854**

(43) Date of publication of application:
**14.06.2023 Bulletin 2023/24**

(73) Proprietor: **Universidad de Granada**
**18071 Granada (ES)**

(72) Inventors:
• **PORRAS SÁNCHEZ, Ignacio**
**18071 Granada (Granada) (ES)**
• **PRAENA RODRÍGUEZ, Antonio Javier**
**18071 Granada (Granada) (ES)**
• **ARIAS DE SAAVEDRA ALÍAS, Fernando**
**18071 Granada (Granada) (ES)**
• **TORRES SÁNCHEZ, Pablo**
**18071 Granada (Granada) (ES)**
• **RAMOS CHERNENKO, Nataliya**
**18071 Granada (Granada) (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(56) References cited:
EP-A1- 1 895 819         EP-A1- 3 254 729
EP-A1- 3 453 428         WO-A1-2019/114307
WO-A1-2019/114307    WO-A1-2020/101858
CN-A- 108 355 257       US-A1- 2020 147 414

• FATEMEH S RASOULI ET AL: "Design of a model for BSA to meet free beam parameters for BNCT based on multiplier system for DT neutron source", ANNALS OF NUCLEAR ENERGY, PERGAMON PRESS, OXFORD, GB, vol. 39, no. 1, 30 August 2011 (2011-08-30), pages 18 - 25, XP028103089, ISSN: 0306-4549, [retrieved on 20110917], DOI: 10.1016/J.ANUCENE.2011.08.025
• BLEUEL D L ET AL: "DESIGNING ACCELERATOR-BASED EPITHERMAL NEUTRON BEAMS FOR BORON NEUTRON CAPTURE THERAPY", MEDICAL PHYSICS, AIP, MELVILLE, NY, US, vol. 25, no. 9, 1 September 1998 (1998-09-01), pages 1725 - 1734, XP000835232, ISSN: 0094-2405, DOI: 10.1118/1.598353

- **FATEMEH S RASOULI ET AL.: "Design of a model for BSA to meet free beam parameters for BNCT based on multiplier system for DT neutron source", ANNALS OF NUCLEAR ENERGY, vol. 39, no. 1, 30 August 2011 (2011-08-30), OXFORD, GB, pages 18 - 25, XP028103089, ISSN: 0306-4549, [retrieved on 20211124], DOI: 10.1016/ j.anucene. 2011.08.02 5**

**Description**

**FIELD OF THE INVENTION**

The present invention relates generally to a device for the production, moderation and configuration of beams, and more specifically neutron beams for neutron capture therapies.

**BACKGROUND OF THE INVENTION**

Boron neutron capture therapy (commonly abbreviated as BNCT) is a type of experimental cancer radiotherapy which is unique in that it is selective at the cellular level, such that the dose applied to healthy tissue and tumor tissue can be controlled in a differentiated manner. In this way, tumor cells can be killed without damaging the surrounding healthy tissue. BNCT techniques are mainly based on irradiating the patient, who has previously been administered a boron compound (since $^{10}B$ has a high probability of being captured by neutrons), with neutrons. Generally, said neutron beam is generated by means of a nuclear reactor or more recently using a particle accelerator. Boron preferably binds to cancer cells such that, when a neutron interacts with it, a nuclear reaction which kills or severely damages the cancer cell occurs, leaving the cells of adjacent tissue with hardly any damage.

To be able to put boron neutron capture therapies into practice, there is a need to have a neutron beam with specific qualities. Said neutron beam must be of the epithermal type (that is, with energies comprised between 0.5 eV and 10 keV) and have an intensity adapted to the type of tumor to be treated. Furthermore, the beam must exhibit a low contamination of thermal neutrons (energies below 0.5 eV) and rapid neutrons (energies above 10 keV), as well as of gamma-radiation. Likewise, the treatment must take into account epithermal neutron thermalization effect (i.e., the process through which the energy of said neutrons decreases as they penetrate the tissue, reaching a thermal peak between 3 and 6 cm of depth). Furthermore, the neutron beam is required to have a low divergence as this allows maximizing the radiation dose to the tumor, while the rest of the patient's body receives the lowest possible dose. These conditions have been standardized in technical document 1223 of the International Atomic Energy Agency (IAEA) (see D. Rorer et al., "Current Status of Neutron Capture Therapy, IAEA TECDOC 1223. ", International Atomic Energy Agency, Vienna, 2011).

In the state of the art (for example, [I. Porras et al., "Perspectives on Neutron Capture Therapy of Cancer", CERN Proc., 1, 295-304, 2019] and in [Torres-Sánchez et al., "On the upper limit for the energy of epithermal neutrons for Boron Neutron Capture Therapy", Radiation Physics and Chemistry, 156, 240-244, 2019]) it has been disclosed that the optimal neutron energy for BNCT treatment is in the range of a few keV (1-10 keV), depending on the tissue on which it is applied. Furthermore, to ensure the applicability of neutron beam in BNCT treatment, it is recommended to measure, among others, the following quality factors relating to radiation tissue penetration capacity:

- Advantage depth (better known as AD): this is defined as the depth in tissue at which the radiation dose received by the tumor is equal to the maximum dose deposited in healthy tissue. This parameter determines the recommended maximum application depth of BNCT treatment.
- Treatable depth (TD): this indicates the depth at which the dose to the tumor tissue is twice the maximum dose deposited in healthy tissue. It therefore determines the region where BNCT treatment can be most effective.
- Advantage depth dose rate ("ADDR): this is equivalent to the maximum dose rate in healthy tissue. If the treatment time is fixed, it allows knowing the maximum dose received by healthy tissue.
- Treatment ratio (TR): this is given by the ratio between the maximum dose in the tumor and the maximum dose in healthy tissue. It is therefore a parameter that should be maximized to prevent damages to healthy tissue.

The IAEA also defines another series of recommendations with respect to parameters relating to the energy of the generated neutrons:

- Epithermal flux ($\phi_{epi}$): this measures the epithermal neutron flux bombarded to the tissue. The recommendation indicates that this parameter must be greater than $10^9$ epithermal neutrons (n) per square centimeter per second (units: $n/cm^2 s$).
- Ratio between thermal and epithermal neutrons: $\phi_{th}/\phi_{epi}$, where $\phi_{th}$ is the thermal neutron flux, which must be below 0.05 expressed on a per unit basis.
- Ratio between total current ($J_n$) and total flux: $J_n/\phi_n$, which is related to the direction and divergence of the beam produced. The greater this parameter is, the better the beam focuses and the less healthy tissue in the vicinity of the tumor tissue is irradiated. It is desirable for this parameter to be greater than 0.7.
- Fast neutron dose ($D_{fast}$) per epithermal neutron: It is desirable to minimize this parameter to below $2 \cdot 10^{-13} Gy \cdot cm^2/n$.
- Gamma-radiation dose ($D_{\gamma}$) per epithermal neutron: like the foregoing, it is desirable to minimize this dose in order to reduce the radiation received by the patient. The value thereof must be below $2 \cdot 10^{-13} Gy \cdot cm^2/n$.

Simulations with tissue reference models such as, for example, the ICRU4 standard tissue phantom (recommended by the "International Commission on Radiation Units and Measurements") and the Snyder brain model (see W.S. Snyder et al., "Estimates of absorbed fractions for monoenergetic photon sources uniformly distributed in various organs of a heterogeneous phantom", Oak Ridge National Lab, Tenn., J. Nucl. Med. 10: Suppl. No. 3, 7-5,1969) are often used to compare the different BNCT treatment devices.

In the past, BNCT was performed using neutron sources from nuclear reactors. As a reference, the FiR-1 facilities (a nuclear reactor located in Helsinki, currently dismantled) and the KURRI facility in Kyoto should be mentioned. However, today and as a result of the development of low-energy and high-intensity accelerator technology, BNCT treatments can be carried out in a hospital setting (see Brugger, R. M., et al. "Rapporteurs' report. Neutron beam design, development, and performance for neutron capture therapy". Springer, Boston, MA, 1990. 3-12). For example, the "Cyclotron Based Epithermal Neutron Source" (abbreviated as C-BENS and located in Kyoto) is a cyclotron accelerator-based BNCT facility for producing an epithermal neutron source and is currently in operation.

In recent years, efforts have been made to search for an optimal neutron beam, with different reactions for the production of neutrons, such as $^7Li(p,n)$, $^9Be(p,n)$ or $^9Be(d,n)$, as disclosed in A. J. Kreiner et al. "Present status of accelerator-based BNCT." Reports of Practical Oncology & Radiotherapy 21.2 (2016): 95-101, being tested. However, the neutrons produced with these reactions have energies exceeding those required in BNCT treatment (of the order of hundreds of keV to MeV). There is therefore a need for a device for the production, moderation and configuration (DPMC) of a neutron beam for neutron capture therapy which adapts the neutrons produced to treatment needs and is capable of adequately meeting all IAEA recommendations considering exclusively the epithermal range of neutrons, i.e., 0.5 eV-10 keV, which is the most suitable for BNCT treatments.

EP 1 895 819 A1 discloses a device for the production, moderation and configuration of a neutron beam, based on the production of neutrons from a proton beam, the device comprising:

- an inlet opening through which the proton beam is directed
- target arranged in the path of direction of the proton beam in order to nuclearly interact with said beam and produce a neutron beam which defines a main axis of propagation;
- a cooling module for cooling the target;
- a moderator contiguous to the target, with a thickness from the target and in the direction of the beam to a filtration stage,
- a reflective cover surrounding the moderator to redirect diverted neutrons to the main axis and increase the epithermal neutron flux;
- an outlet opening through which the neutron beam exits;
- a shield arranged around the outlet opening to suppress the neutrons and gamma-radiation that do not exit the device via said outlet opening; and
- a filtration stage contiguous to the outlet opening and also surrounded by the reflective cover, to filter the neutron beam before it exits the device via the said outlet opening.

## BRIEF DESCRIPTION OF THE INVENTION

As mentioned in the preceding section, there is a need, in the field corresponding to devices for the production, moderation and configuration (DPMC) of neutron beams, to develop devices capable of meeting all the International Atomic Energy Agency (IAEA) recommendations considering exclusively neutrons in the epithermal range (0.5 eV- 10 keV). The present invention provides a solution to said need by means of a device capable of providing ranges of energy that are more suitable and safer for use in boron neutron capture treatments.

More specifically, the main object of the present invention relates to a DPMC of neutron beams based on the production of neutrons from proton beams, which allows obtaining a neutron flux above 109 n/(s cm2) between 1 eV and 20 keV, keeping (i) the thermal neutron flux below 5% of the total flux, (ii) the dose produced by the rapid neutrons below 2 10-13 n/(s cm2) per neutron between 1 eV and 20 keV, and (iii) the dose produced by the secondary photons below 2 10-13 n/(s cm2) per neutron between 1 eV and 20 keV.

This DPMC of neutron beams based on the production of neutrons from proton beams according to the invention is as defined in the appended claims.

Said DPMC is characterized in that the filtration stage acts on the neutron beam before it exits the device through the outlet opening. The neutron beam obtained can be applied, for example, on patient's tissues.

In a preferred embodiment of the invention, the proton beam striking the inlet opening is obtained by means of a particle accelerator, said proton beam being accelerated against a target, manufactured from a metal material, in order to generate neutrons.

In another preferred embodiment, the target comprises $^7$Li and the nuclear reaction with the incident proton beam is $^7$Li(p,n)$^7$Be.

In particular embodiments, the moderator comprises at least one of the following materials: graphite, $D_2O$, $AlF_3$, $CaF_2$, $Li_2CO_3$, $MgF_2$, $Al_2O_3$, Fe. In particular, if the energy source (proton beam) is a low-energy source, the use of $MgF_2$ is preferred. If the source is a high-energy source, then it is preferable to gradually replace $MgF_2$ with $AlF_3$, and replace both in turn with $CaF_2$ and Fe.

In other particular embodiments, the moderator comprises at least one section with one of the following geometries: cylindrical, conical, prismatic, truncated pyramid.

In some preferred embodiments of the invention, the reflective cover comprises at least one of the following materials: Ni, Pb, BeO, Bi.

In some advantageous embodiments of the invention, the rapid neutron filtration layer of the filtration stage comprises at least one of the following materials: Al, Fe, Ni.

In other particular embodiments of the invention, the thermal neutron filtration layer of the filtration stage comprises at least one of the following materials: $^{10}$B, $^6$Li, Gd, Cd, LiF. Excess thermal radiation that healthy surface tissue would receive during the application of the neutron beam is thereby reduced. Thermal neutrons are not useful for application in tissues because they hardly penetrate deeply into these tissues, such that they are only applicable if used on surface or shallow injuries. Gd and Cd are among the materials having the largest thermal capture cross-sections, so the use thereof to remove thermal neutrons would be optimal if it is not required to minimize the gamma-radiation production in the same way. In both cases, gamma-radiation production is significant, which would imply a greater thickness of Pb or Bi as a gamma-radiation filter. Alternatively, this filtration layer can be placed in a part prior to moderation, such that gamma-radiation would be partially attenuated by the passage of the rest of the materials, but in such a case, the effect thereof as a thermal neutron attenuator would be reduced.

In other preferred embodiments of the invention, the gamma-radiation filtration layer of the filtration stage comprises at least one of the following materials: Pb, Bi. In this way, contamination of the neutron beam obtained with the invention by gamma-radiation is reduced.

As a result of the three aforementioned layers which the filtration stage of the invention at least comprises, radiation sources which are not epithermal neutrons are reduced. This gives the spectrum of the generated neutron beam a high energy selectivity, having maximums in the range of 2-3 keV, and being highly suitable for neutron capture treatment, and more specifically BNCT.

In some particular embodiments of the invention, the outlet opening for the neutron beam comprises at least one section with one of the following geometries: cylindrical, conical, prismatic, truncated pyramid. In this way, the divergence of the neutron beam obtained is reduced.

In certain advantageous embodiments of the invention, the outlet opening (6) for the neutron beam comprises a movable closure to stop irradiation.

In other embodiments of the invention, the shield arranged around the outlet opening for the neutron beam comprises at least one of the following materials: LiF, $^6$LiF, $B_4$C, polyethylene, Pb, Bi. In other preferred embodiments, the shield only comprises Pb and lithiated polyethylene. In some even more advantageous embodiments, two additional layers of natural LiF and $^6$Li-enriched LiF are added to the Pb layer and to the lithiated polyethylene layer. As a result of the shield, most neutrons diverting from the main axis and the gamma-radiation associated therewith can thus be suppressed, in order to reduce the radiation dose received by normal tissue or regions close to the irradiated tissue, in which the residual radiation received should the lowest possible. $B_4$C is the commonly used material as it has $^{10}$B and is solid.

The preferred use of the DPMC of the neutron beam of the invention consists of boron neutron capture therapy. As a result of the filtration stage, prior to the exit of the neutron beam of the invention, said neutron beam has a spectrum suitable for use thereof in this type of therapy.

Throughout the text, the word "comprises" (and its derivatives) should not be understood in an exclusive manner, but rather in the sense of allowing the possibility that what is defined may include additional elements or steps.

## DESCRIPTION OF THE FIGURES

A set of figures which are integral part of the description and illustrate a preferred embodiment of the invention is provided to complete the description of the invention. Said figures should be interpreted in a non-limiting illustrative manner and are described in detail below.

Figure 1 shows the preferred implementation of the invention by means of a two-dimensional axial section in which the main dimensions of the device, as well as the materials making up same, are indicated.

Figure 2 illustrates a three-dimensional view of the invention shown in Figure 1, with a radial section to enable viewing the distribution of materials and layers in the inside thereof.

Figure 3 shows the spectrum of neutrons at different points of the invention, from the Li source to the outlet opening, in order to demonstrate how the suitable selection of materials minimizes the thermal neutron and rapid neutron fluxes without reducing epithermal neutrons and reaching spectral maximum at 2-3 keV. The epithermal range (0.5 eV-10 keV) is indicated in the shaded region.

Figure 4 depicts, in logarithmic scale, the final spectrum of the radiation obtained in the outlet opening of the invention, in comparison with two of the reference BNCT treatment facilities: FiR-1 in Helsinki (Finland) and C-BENS in Kyoto (Japan). The epithermal range (0.5 eV-10 keV) is indicated in the shaded region.

Figure 5 illustrates, in linear scale, the final spectrum of the radiation obtained in the outlet opening of the invention, in comparison with the same facilities of Figure 4. The epithermal range (0.5 eV-10 keV) is indicated in the shaded region.

Figure 6 shows, in logarithmic scale, a lateral profile of the (epithermal, thermal and rapid) neutron flux and that of gamma-radiation, in the radial direction right at the outlet opening of the invention.

A series of reference numbers, corresponding to the following elements, accompany the mentioned figures:

(1) Inlet opening
(2) Target
(3) Moderator
(4) Reflective cover
(5) Filtration stage
(6) Outlet opening
(7) Shield
(W1) Thickness of air in the direction of the beam, from the last filtration layer (5) to the inlet opening (1).
(W2) Thickness of the moderator (3) in the direction of the beam, from the target (2) to the filtration stage (5).
(L1) Thickness of the rapid neutron filtration layer
(L2) Thickness of the thermal neutron filtration layer
(L3) Thickness of the gamma-radiation filtration layer
(L4) Thickness of the anterior or front part of the moderator in the embodiments in which the diameter thereof is reduced as it approaches the outlet opening.
(L5) Thickness of the rear part of the moderator in which the diameter (Ø3) thereof remains constant.
(L6) Thickness of the cover (4) from the moderator (3) to the inlet opening, in the direction of the beam.
(Ø2) Diameter of the rear part of the moderator
(Ø3) Diameter of the cover (4)
(Ø4) Diameter of the device, including the side shield (7)
(R1) Inner radius of the collimator of the front shield (7)
(R2) Outer radius of the collimator of the front shield (7)

## DETAILED DESCRIPTION OF THE INVENTION

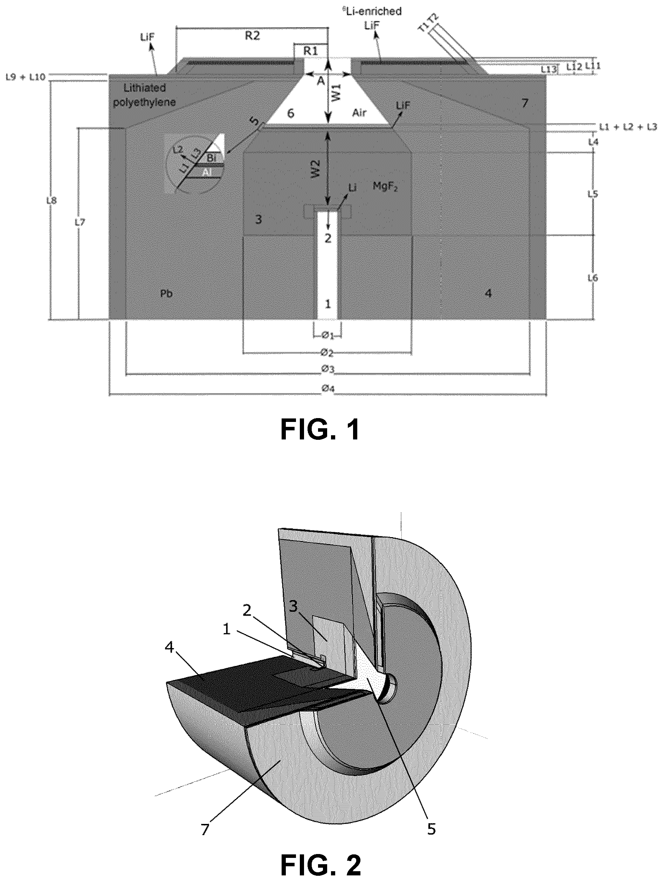

Figure 1 shows a preferred implementation of the invention which has axial symmetry and a cylindrical-conical geometry. First, it is required to accelerate a proton beam by means of an accelerator (not shown in the figure) up to 2.1 MeV (however, other values can also be used), and by directing said proton beam through the inlet opening (1) or a directing tube of the invention, all the IAEA recommendations are met. At this energy of the proton beam, the neutrons generated by the invention have a mean energy of 108.4 keV and a maximum energy of 350.4 keV. Next, a proton beam bombards a target (2) which, in this case, is a lithium sheet ($^7$Li) located at the end of the directing tube of the accelerator, and neutrons are produced by means of a nuclear reaction $^7$Li(p,n)$^7$Be. Said proton beam is oriented according to a main axis. However, other preferred embodiments of the invention may involve different nuclear reactions to generate neutrons, for example, $^9$Be(p,n). The invention also comprises a cooling module for cooling the target (2) which is not shown in the figures but is necessary in order to prevent said target (2) from melting due to the high temperatures that can be reached.
Surrounding the lithium target (2) and extending in the front direction and to the sides, there is placed the core of the moderator (3) with a thickness (W2) in said front direction, from the target (2) to the filter (5), of 21.80 cm $\pm 10\%$, preferably 21.8 cm. Preferably, the moderator will be manufactured with $MgF_2$. Other materials which can be used as the moderator (3) are combinations of Al, Mg or Ca metals with F, as well as carbon in the form of graphite, or water ($H_2O$ or heavy water, $D_2O$). These materials are chosen based on their neutron interaction properties. Therefore, isotopes having a large elastic cross-section and a small absorption cross-section are required. Neutrons can lose energy gradually by means of elastic collisions. This energy loss by collision is greater for lightweight isotopes, such as hydrogen or deuterium, or carbon. The suitability of the elements Al, Mg, Ca or F is mainly due to their energy resonances of the range of tens to hundreds of keV, which allows reducing the amount of high-energy neutrons which transition to the epithermal range through moderation. $MgF_2$ is chosen over other materials due to its larger elastic cross-section, while the production of gamma-radiation per capture is lower than in other materials such as water. Furthermore, the nuclei are not as lightweight as to lose a large proportion of energy in each collision, which allows the neutrons to lose their energy much more gradually and controllably. Other metals such as Al or Ca have higher energy resonances, so the use thereof as low-energy sources is not as suitable as Mg. In other preferred implementations of the invention, the moderator can be manufactured with at least one of the following materials: $D_2O$, $Al_2O_3$, $Li_2CO_3$, or others having equivalent or similar properties known by one skilled in the art. In Figure 1, the moderator (3) includes a cylindrical section and another conical section. However, in other preferred embodiments the moderator (3) comprises at least one section or profile with one of the following geometries: cylindrical, prismatic, truncated pyramid, conical.

Preferably, there is arranged around the moderator (3) a reflective cover (4), made with a material with a large elastic collision cross-section and at the same time a high mass number, such that the neutrons do not lose more energy in these collisions, and furthermore the probability of backward collision is higher, which allows neutrons to be recovered towards the core of the moderator. The reflector therefore redirects the diverted neutrons to the main axis of the neutron beam, which increases the epithermal neutron flux. In its rear part, from the moderator to the inlet opening, the cover (4) has a thickness (L6) of 25.00 cm $\pm 40\%$ in the direction of the beam.

The moderator also covers the target and part of the tube of the accelerator to the rear part (beam inlet). In a preferred embodiment, the total thickness of the moderator (L4+L5) is comprised between 24.64 and 36.96 cm.

Furthermore, the material of the reflective cover (4) must absorb the gamma-radiation generated in the moderation process. For this reason, materials with a high atomic number are ideal for this task. In this way, the optimal materials for this function are lead and bismuth. Pb has an elastic collision cross-section that is larger than Bi, such that it has been used as a reflector in general, except in the front direction, where the divergence of the beam is a factor to be considered. In the radial as well as rearward directions, increasing the thickness (L6) of the Pb layer allows recovering more neutrons which have escaped the core of the moderator (3). This relationship is maintained until a maximum thickness, at which the increase in epithermal neutrons becomes saturated, is reached.

A filtration stage (5), also surrounded by the reflective cover (4), is arranged after the core of the moderator (3). Said filtration stage (5) functions to provide an additional filtration of the spectrum of neutrons which do not contribute to BNCT treatment (rapid and thermal neutrons), as well as to reduce the gamma-radiation minimally affecting the epithermal neutron flux. Preferably, the mentioned filtration stage (5) comprises at least these layers:

- Rapid neutron filtration layer: this layer complements moderation and fundamentally performs the function of filtering the spectrum of neutrons obtained by selectively removing some rapid neutrons due to their resonances. The thickness of this layer (L1) is 1.00 cm $\pm 15\%$. In Figure 1, this layer consists of an aluminum sheet and its thickness is 1 cm. However, other elements such as Fe or Ni may be suitable.
- Thermal neutron filtration layer: the purpose of this layer is to suppress the thermal neutrons of the beam. The moderation process subsequently produces a considerable amount of thermal neutrons which must be removed from the beam prior to the use thereof in BNCT treatment. Li is a material with a large thermal capture cross-section. This characteristic makes it an optimal thermal neutron filter.

The thickness of this layer (L2) is 0.20 cm $\pm 20\%$. In particular, in the implementation of Figure 1, this layer is a LiF sheet of only 2 mm thick but allows removing more than half of the thermal neutron flux reaching it. Other materials which can also be used for this thermal neutron filtration layer are B and Cd which, though having larger capture cross-sections, in exchange also generate gamma-radiation and require a larger amount of Pb or Bi in the reflective cover (4) in order to remove same.

- Gamma-radiation filtration layer: this layer is designed for mitigate gamma-radiation but by affecting the epithermal neutron flux as little as possible. The thickness of this layer (L3) is 1.00 cm $\pm 10\%$. In Figure 1, this layer consists of a Bi sheet of 1 cm thick, which allows obtaining a greater neutron flux with a lower divergence.

Preferably, the gamma-radiation filtration layer is arranged last according to the direction of travel of the neutron beam produced. Even more preferably, the thermal neutron filtration layer and the gamma-radiation filtration layer are arranged in that order according to the direction of travel of the neutron beam produced.

It can be seen in Figure 1 that the filtration stage (5) comprises the aluminum, LiF and Bi layers in said order according to the direction of travel of the neutron beam produced. However, the mentioned layers can be arranged in any technically possible order, and can even be interchanged with part of the block of the moderator. Other preferred embodiments of the invention comprises a plurality of one or more of the layers comprised in the filtration stage (5).

As a result of the filtration stage (5), the spectrum of the neutrons obtained is suitable for BNCT treatment (the rapid and thermal neutrons have been filtered, allowing the thermal neutron flux to be kept below 5% of the total flux, and also residual gamma-radiation has been removed).

Next there is arranged an outlet opening (6) which determines the shape and focalization of the beam. In particular, it has a cylindrical-conical section, as shown in Figure 1. To that end, said opening comprises a collimation cone, also preferably made with Pb, which makes it possible to shape the neutron beam taking into account the inclination of its generatrix. A too large inclination (i.e., a very elongated collimation cone) reduces the epithermal flux too much. In contrast, a very low inclination (i.e., a very flattened cone) generates excessive divergence of the beam. The neutron beam that can be used for BNCT treatment exits through said outlet opening (6), filled with air. It should be noted that in some preferred implementations of the invention, the outlet opening (6) comprises slits and/or projections. Likewise, in other advantageous implementations the opening (6) comprises a movable closure (also known as beam shutter) that can be remotely actuated to stop irradiation, comprising a doble Li and Pb layer.

Finally there are arranged various layers of shield (7) which act as protection of the patient against gamma-radiation, as well as absorb the neutrons which would otherwise exit somewhere other than via the outlet opening (6). After the outlet opening (6) there are placed various materials which make it possible to absorb the neutrons which would otherwise exit the invention in an uncontrolled manner. As shown in Figure 1, this shield (7) comprises at least two layers, one of each of the following materials:

- Lithiated polyethylene, comprising $^6$Li-enriched polyethylene to absorb thermal neutrons, as a coating for the reflective cover (4). As said polyethylene contains hydrogen, it allows the energy of the neutrons to be rapidly reduced to the thermal range, while the $^6$Li captures them without generating gamma-radiation. As a result of this layer, the neutrons that may exit in these directions are thermalized and absorbed as an additional radiation protection measure.
- Lead, to absorb gamma-radiation.

The shield (7) may contain only the above two layers, the Pb layer and the lithiated polyethylene layer, in case a mixture with a sufficiently high proportion of $^6$Li is achieved. Alternatively, the following two layers can be used additionally to increase the concentration of the $^6$Li isotope: a natural LiF layer and a $^6$Li-enriched LiF layer, which together with the natural LiF layer prevent contamination of the neutron beam obtained with contributions off the main axis.

In different preferred implementations of the invention, there can be a plurality of one or more layers of the shield (7) of the aforementioned materials: lithiated polyethylene, lead, LiF, $^6$Li-enriched LiF. In other implementations, the lithiated polyethylene can be replaced with borated polyethylene.

The layers of the shield (7) have been described in the sense in which they would be traversed by the neutron beam. However, they can be arranged in any technically possible order. The outlet opening (6) is thus covered by lead and LiF layers in both its conical and cylindrical cross-section. However, it should be noted that the arrangement of layers of the shield (7) shown Figure 1 is only an illustrative example and may vary depending on the design requirements.

In summary, the DPMC must be capable of producing an epithermal neutron beam with high flux, while at the same time thermal and rapid neutron fluxes must be reduced to a minimum. The optimal energy of the neutrons which are applied in BNCT is a few keV, since it allows the treatment of deep tumors, so the maximum of the spectrum must be in this energy range. Likewise, the contamination of the beam by gamma-radiation must be avoided as much as possible. Finally, the neutron beam must have good collimation and a not very high divergence. In other words, the generated neutrons must exit towards the front and the beam must not open up excessively after exiting thorough the outlet opening (6). It should be noted that in the invention there are two different gamma-radiation filters, but their function is different: a layer included in the filtration stage (5), which filters said radiation for the beam that is to exit through the outlet opening (6) and that is to be used in the treatment; while the gamma-radiation filters included in the shield (7) only affect the diverted neutrons with respect to the main axis.

In the preferred embodiment of the invention laid out in Figure 1, the design is generated from two parameters which must previously be imposed: A (14 cm) and Ø1 (8 cm). The rest of the parameters represent the optimal dimensions of the design. However, the DPMC is operative within a certain tolerance range of said parameters, such that the optimal value of each parameter is indicated below, in centimeters, together with the percentage variation allowed without significantly degrading its operation: Ø2 (50, ±10%), Ø3 (120, ±15%), Ø4 (130, ±20%), R1 (10, ±10%), R2 (45, ±50%), W1 (19.80, ±10%), W2 (21.80, ±10%), L1 (1.00, ±15%), L2 (0.20, ±20%), L3 (1.00, ±10%), L4 (6.20, ±10%), L5 (24.60, ±10%), L6 (25.00, ±40%), L7 (57.00, ±20%), L8 (70.80, ±20%), L9 (1.00, ±50%), L10 (1.00, ±50%), L11 (5.00, ±10%), L12 (4.00, ±25%), L13 (3.00, ±25%), T1 (2.00, ±50%), T2 (2.00, ±50%). The size of the outlet opening has been designed with the value A=14 cm. However, a modification A±2δ would entail changes in other related radial dimensions, as follows: Ø2, Ø3 and Ø4 would vary ±2δ (the same as A), while R1 and R2 would vary half as much as A (±δ). In this way, the inlet opening (1) for the beam could be modified according to the directional requirements of the target (2). The design of Figure 1 is also shown in Figure 2, in a three-dimensional view. It should be noted that the device has axial symmetry.

The validation of the preferred embodiment shown in Figures 1 and 2 has been carried out by comparing the quality parameters imposed by the IAEA with those obtained with said design. First, the DPMC has been verified by means of Monte Carlo simulation for a proton beam at 2.0 and 2.1 MeV, respectively, and for two types of phantoms: a cylindrical phantom model filled with ICRU-4 standard tissue and another Snyder phantom model.

The results of the simulations on the phantoms are summarized in Table 1.

**TABLE 1**

| Parameter | ICRU-4 (2.0 MeV) | ICRU-4 (2.1 MeV) | Snyder (2.0 MeV) | Snyder (2.1 MeV) |
|---|---|---|---|---|
| AD (cm) | 9.54 | 9.53 | 9.29 | 9.27 |
| TD (cm) | 7.38 | 7.37 | 7.24 | 7.23 |
| ADDR (Gy-Eq/min) | 0.16 | 0.30 | 0.13 | 0.25 |
| TR | 4.72 | 4.71 | 4.67 | 4.65 |

The following equation has been used to estimate the total radiation equivalent dose ($D_T$), in units of equivalent grays (Gy-Eq.):

$$D_T = (w| \quad |th + \chi r \cdot w_B)D_{th} + w_{fast} \cdot D_{fast} + D \quad ,$$

where $D_{th}$ is the thermal neutron dose, $r = 0.422$ and $\chi$ denotes the concentration of boron applied with the treatment (its value is about 35 in tumor tissue and 10 in healthy tissue); while $w_{th}, w_{fast}$ and $w_B$ are a series of weighting parameters depending on the relative biological effectiveness (RBE) of the contributions of thermal neutrons, rapid neutrons, and boron, respectively. In particular, $w_{th}$ as $w_{fast}$ are equal to 3.2 both for tumor tissue and for healthy tissue. In contrast, the weighting of boron is higher in tumor tissue (where $w_B = 3.8$) in comparison with healthy tissue ($w_B = 1.3$).

Secondly, in Table 2 various quality parameters of the neutron beam obtained in the DPMC are compared based on the IAEA recommendations. In particular, the results for a proton beam at 2.0 and 2.1 MeV, respectively, are shown. The epithermal limit of energy (LE) is set at 10 and 20 keV. It should be noted that about half of the dose produced by rapid neutrons corresponds to neutrons with an energy in the range of 10-20 keV. Furthermore, all the IAEA recommendations are met with the proposed invention when the incident proton beam has an energy of 2.1 MeV, while in the case of 2.0 MeV, it only falls below in terms of epithermal flux. Another aspect to be noted is that as a result of the proposed preferred embodiment, the IAEA requirements can be met considering exclusively epithermal neutrons in the range of 0.5 eV-10 keV, which are the most suitable for BNCT treatment. This is achieved as a result of the filtration stage (5) and the shield (7), as well as the materials forming same.

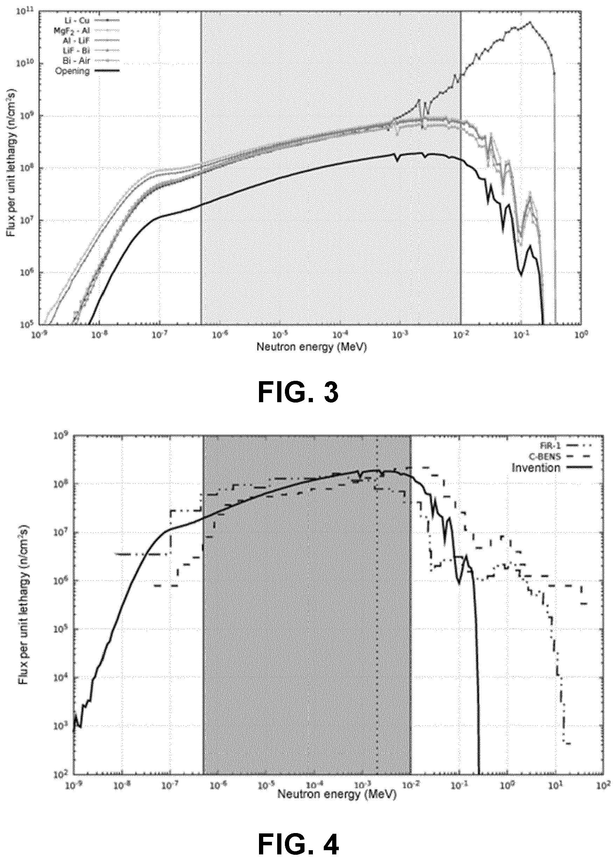

Thirdly, the neutron spectrum in the DPMC has been analyzed. In particular, Figure 3 shows the neutrons spectrum at different positions within the DPMC and at the outlet opening (6) thereof, reflecting the process of moderation and subsequent filtration of unwanted neutrons. The selection of materials of the design allows thermal and rapid electron fluxes to be minimized without reducing epithermal fluxes. Particularly relevant is the moderation of the neutrons with the highest energy in the $MgF_2$ layer with which the spectral maximum is achieved at 2-3 keV, which is optimal for BNCT treatments, especially deep tumors.

**TABLE 2**

| Parameter | IAEA Standard | Invention (at 2.0 MeV) | | Invention (at 2.1 MeV) | |
|---|---|---|---|---|---|
| | | LE 10 keV | LE 20 keV | LE 10 keV | LE 20 keV |
| $\phi_{epi}$ (n/cm$^2$s) | $> 5 \cdot 10^8$ | $5.450 \cdot 10^8$ | $5.785 \cdot 10^8$ | $1.012 \cdot 10^9$ | $1.078 \cdot 10^9$ |
| $\phi_{th}/\phi_{epi}$ | $< 0.05$ | 0.0328 | 0.0309 | 0.0326 | 0.0306 |
| $J_n/\phi_n$ | $> 0.7$ | 0.7116 | 0.7116 | 0.7108 | 0.7108 |
| $D_{fast}$ (Gy·cm$^2$/n) | $< 2 \cdot 10^{-13}$ | $1.81 \cdot 10^{-13}$ | $0.93 \cdot 10^{-13}$ | $1.99 \cdot 10^{-13}$ | $1.05 \cdot 10^{-13}$ |
| $D_\gamma$ (Gy·cm$^2$/n) | $< 2 \cdot 10^{-13}$ | $1.12 \cdot 10^{-13}$ | $1.05 \cdot 10^{-13}$ | $1.12 \cdot 10^{-13}$ | $1.05 \cdot 10^{-13}$ |

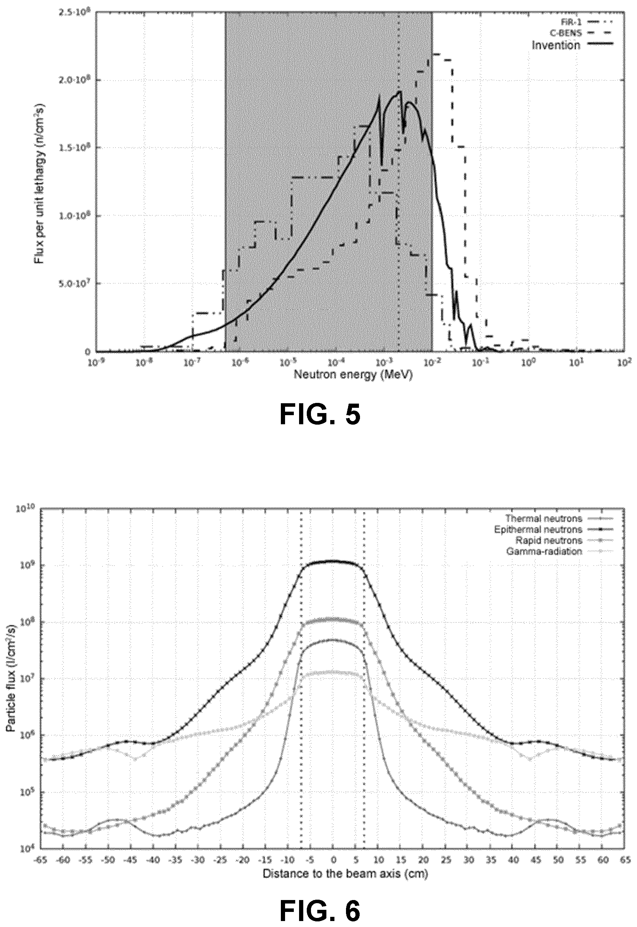

In addition, it has been compared with the spectrum provided by other DPMC facilities, in particular Fir-1 and C-BENS. The results are shown in Figure 4 (in logarithmic scale) and Figure 5 (in linear scale).

Figure 6 shows, in logarithmic scale, the lateral profile of the (epithermal, thermal and rapid) neutron flux and that of gamma-radiation, in the radial direction right at the outlet opening (6). It is remarkable the good definition of the beam at the outlet opening (6), delimited by the dotted vertical dashed lines in the central area of the graph, as well as the efficient suppression of the neutrons outside said outlet opening (6). In particular, the epithermal neutron flux is reduced by two orders of magnitude within the first 15 cm, while for thermal neutrons this attenuation is much more pronounced and occurs in the first 5 cm. In addition, gamma-radiation is well attenuated over the entire irradiation area. This avoids contamination of the neutron beam obtained, both in terms of gamma-radiation and the contribution off the main axis.

In some preferred embodiments of the invention, the reflective cover (4) comprises at least one of the following materials: Ni, Pb, BeO, Bi. In the case of beryllium oxide, the usable isotope is $^9$Be. This has a high elastic cross-section, which constitutes almost the entirety of its cross-section up to the MeV range, so that neutron absorption losses would be minimal in this case. However, it has two drawbacks, both due to the low mass of $^9$Be. On the one hand, neutron collisions do not occur predominantly at large angles (backward bouncing), so many neutrons will follow their path to the outside of the DPMC, slightly diverted and not reflected, thus requiring a larger thickness of material. On the other hand, the kinematics of the elastic collision cause the neutrons to lose much more energy compared to a heavier nucleus, so the neutrons would be excessively moderated. Thus, BeO might be more appropriate in the case of using higher initial energies for the neutrons, beyond the 2-2.1 MeV used in this embodiment of the invention. In such a case it would contribute to moderation together with $MgF_2$, as a result of its intermediate behavior between a reflector and a moderator.

## Claims

1. A device for the production, moderation and configuration of a neutron beam, based on the production of neutrons from a proton beam obtained by means of a particle accelerator, comprising:

   - an inlet opening (1) through which the proton beam is directed;
   - a target (2) arranged in the path of direction of the proton beam in order to nuclearly interact with said beam and produce a neutron beam which defines a main axis of propagation;
   - a cooling module for cooling the target (2);
   - a moderator (3) contiguous to the target (2), with a thickness (W2) from the end of the target (2) and in the direction of the beam to the filtration stage (5), of between 19.62 and 23.98 cm, where the neutrons produced in the nuclear interaction are moderated to energies of the epithermal range
   wherein the total thickness of the moderator (L4+L5) is comprised between 24.64 and 36.96 cm, covering the target (2) and part of the tube of the particle accelerator to the inlet opening (1),
   - a reflective cover (4) surrounding the moderator (3) to redirect diverted neutrons to the main axis and increase the epithermal neutron flux;
   - an outlet opening (6) through which the neutron beam exits; and
   - a shield (7) arranged around the outlet opening (6) to suppress the neutrons and gamma-radiation that do not exit the device via said outlet opening (6); and
   - a filtration stage (5) contiguous to the moderator (3) and contiguous to the outlet opening (6) and also surrounded by the reflective cover (4), to filter the neutron beam before it exits the device via the said outlet opening (6); where said filtration stage (5) comprises at least three layers: a rapid neutron filtration layer, a thermal neutron filtration layer and a gamma-radiation filtration layer.

2. The device for the production, moderation and configuration of a neutron beam according to any of the preceding claims, wherein the rapid neutron filtration layer comprised in the filtration stage has a thickness (L1) of 1.00 cm $\pm$15%.

3. The device for the production, moderation and configuration of a neutron beam according to any of the preceding claims, wherein the thermal neutron filtration layer comprised in the filtration stage (5) has a thickness (L2) of 0.20 cm $\pm$20%.

4. The device for the production, moderation and configuration of a neutron beam according to any of the preceding claims, wherein the gamma-radiation filtration layer comprised in the filtration stage (5) has a thickness (L3) of 1.00 cm $\pm$15%.

5. The device according to any of claims 2 to 4, **characterized in that** the rapid neutron filtration layer comprises Al, Fe or Ni.

6. The device according to the preceding claim, **characterized in that** the rapid neutron filtration layer is manufactured with Al.

7. The device according to any of claims 2 to 6, **characterized in that** the thermal neutron filtration layer comprises $^{10}B$, $^{6}Li$, Gd, Cd or LiF, preferably wherein thermal neutron filtration layer is manufactured with LiF.

8. The device for the production, moderation and configuration of a neutron beam according to any of the preceding claims, wherein the reflective cover (4) surrounding the moderator (3) to redirect diverted neutrons to the main axis, has a thickness (L6) of 25.00 cm $\pm40\%$ from the moderator to the inlet opening, in the direction of the beam.

9. The device according to any of the preceding claims, wherein the reflective cover (4) comprises at least one of the following materials: Ni, Pb, BeO or Bi, preferably wherein the reflective cover (4) is manufactured with Pb.

10. The device according to any of the preceding claims with an outlet opening having a diameter $A\pm2\delta$, having the following radial dimensions:

Diameter (Ø2) of the rear part of the moderator: $A\pm2\delta+36$ cm $\pm10\%$
Diameter (Ø3) of the cover (4): $A\pm2\delta+106$ cm $\pm15\%$
Diameter (Ø4) of the device: $A\pm2\delta+116$ cm $\pm20\%$
Inner radius (R1) of the collimator of the front shield (7): $(0.5A\pm\delta+3$ cm $\pm10\%)$
Outer radius (R2) of the collimator of the front shield (7): $(0.5A\pm\delta+22.5$ cm $\pm50\%)$.

11. The device according to any of the preceding claims, wherein the target (2) comprises $^{7}Li$ and the nuclear interaction with the incident proton beam is $^{7}Li(p,n)^{7}Be$.

12. The device according to any of the preceding claims, wherein the outlet opening (6) for the neutron beam comprises at least one section with one of the following geometries: cylindrical, conical, prismatic, truncated pyramid.

13. The device according to any of the preceding claims, wherein the outlet opening (6) for the neutron beam comprises a movable closure to stop irradiation.

14. The device according to any of the preceding claims, wherein the shield (7) arranged around the outlet opening (6) for the neutron beam comprises at least one of the following materials: LiF, $^{6}LiF$, $B_4C$, polyethylene, Pb, Bi.

15. The device according to any of the preceding claims, for use thereof in boron neutron capture therapy.


**Patentansprüche**

1. Vorrichtung zur Erzeugung, Moderation und Konfiguration von einem Neutronenstrahl, basierend auf der Erzeugung von Neutronen aus einem Protonenstrahl erhalten mittels eines Teilchenbeschleunigers, umfassend:

- eine Eingangsöffnung (1) durch welche der Protonenstrahl gerichtet wird;
- einen Auffänger (2), welcher im Richtungspfad des Protonenstrahls angeordnet ist, um mit dem genannten Strahl nuklear zusammenzuwirken und einen Neutronenstrahl zu erzeugen, welcher eine Hauptausbreitungsachse definiert;
- ein Kühlmodul zum kühlen des Auffängers (2);
- einen Moderator (3), welcher dem Auffänger (2) benachbart ist, mit einer Dicke (W2) vom Ende des Auffängers (2) und in Richtung des Strahls bis zur Filtrierungsphase (5), von zwischen 19,62 und 23,98 cm, in welchem die in der nuklearen Zusammenwirkung erzeugten Neutronen auf Energien des epithermalen Bereichs moderiert werden
wobei die Gesamtdicke des Moderators (L4+L5) zwischen 24,64 und 36,96 cm liegt, sodass er den Auffänger (2) und einen Teil des Rohrs des Beschleunigers zur Eingangsöffnung (1) deckt,
- eine reflektierende Abdeckung (4), welche den Moderator (3) umgibt, um abgelenkte Neutronen zur Hauptachse umzuleiten und den epithermalen Neutronenfluss zu erhöhen;
- eine Ausgangsöffnung (6) durch welche der Neutronenstrahl ausgeht; und
- eine Abschirmung (7), welche um die Ausgangsöffnung (6) herum angeordnet ist, um die Neutronen und die Gammastrahlung, welche nicht aus der Vorrichtung über die genannte Ausgangsöffnung (6) ausgehen, zu unterdrücken; und
- eine Filtrierungsphase (5), welche dem Moderator (3) benachbart ist und der Ausgangsöffnung (6) benachbart ist und auch von der reflektierenden Abdeckung (4) umgeben ist, um den Neutronenstrahl zu filtrieren, bevor er aus der Vorrichtung über die genannte Ausgangsöffnung (6) ausgeht; wobei die genannte Filtrierungsphase (5) mindestens drei Schichten umfasst: eine schnelle Neutronenfiltrierungsschicht, eine thermische Neutronenfiltrierungsschicht und eine Gammastrahlungsfiltrierungsschicht.

2. Vorrichtung zur Erzeugung, Moderation und Konfiguration von einem Neutronenstrahl nach einem der vorhergehenden Ansprüche, wobei die in der Filtrierungsphase umfasste schnelle Neutronenfiltrierungsschicht eine Dicke (L1) von 1,00 cm $\pm$15% aufweist.

3. Vorrichtung zur Erzeugung, Moderation und Konfiguration von einem Neutronenstrahl nach einem der vorhergehenden Ansprüche, wobei die in der Filtrierungsphase (5) umfassten thermische Neutronenfiltrierungsschicht eine Dicke (L2) von 0,20 cm $\pm$20% aufweist.

4. Vorrichtung zur Erzeugung, Moderation und Konfiguration von einem Neutronenstrahl nach einem der vorhergehenden Ansprüche, wobei die in der Filtrierungsphase (5) umfassten Gammastrahlungsfiltrierungsschicht eine Dicke (L3) von 1,00 cm $\pm$15% aufweist.

5. Vorrichtung nach einem der Ansprüche 2 bis 4, **dadurch gekennzeichnet, dass** die schnelle Neutronenfiltrierungsschicht Al, Fe oder Ni umfasst.

6. Vorrichtung nach dem vorhergehenden Anspruch, **dadurch gekennzeichnet, dass** die schnelle Neutronenfiltrierungsschicht mit Al gefertigt wird.

7. Vorrichtung nach einem der Ansprüche 2 bis 6, **dadurch gekennzeichnet, dass** die thermische Neutronenfiltrie- rungsschicht $^{10}$B, $^6$Li, Gd, Cd oder LiF umfasst, wobei vorzugsweise die thermische Neutronenfiltrierungsschicht mit LiF gefertigt ist.

8. Vorrichtung zur Erzeugung, Moderation und Konfiguration von einem Neutronenstrahl nach einem der vorhergehenden Ansprüche, wobei die reflektierende Abdeckung (4), welche den Moderator (3) umgibt, um abgelenkte Neutronen zur Hauptachse umzuleiten, eine Dicke (L6) von 25,00 cm $\pm$40% vom Moderator zur Eingangsöffnung, in Richtung des Strahls, aufweist.

9. Vorrichtung nach einem der vorhergehenden Ansprüche, wobei die reflektierende Abdeckung (4) mindestens eins der folgenden Materialien umfasst: Ni, Pb, BeO oder Bi, wobei vorzugsweise die reflektierende Abdeckung (4) mit Pb gefertigt ist.

10. Vorrichtung nach einem der vorhergehenden Ansprüche mit einer Ausgangsöffnung aufweisend einen Durchmesser A$\pm$2$\delta$, welcher die folgenden Radialabmessungen aufweist:

Diameter (Ø2) des Hinterteils des Moderators: A$\pm$2$\delta$+36 cm $\pm$10%
Diameter (Ø3) der Abdeckung (4): A$\pm$2$\delta$+106 cm $\pm$15%
Diameter (Ø4) der Vorrichtung: A$\pm$2$\delta$+116 cm $\pm$20%
Innenradius (R1) des Kollimators der vorderen Abschirmung (7): (0,5A$\pm$$\delta$+3 cm $\pm$10%)
Außenradius (R2) des Kollimators der vorderen Abschirmung (7): (0,5A$\pm$$\delta$+22.5 cm $\pm$50%).

11. Vorrichtung nach einem der vorhergehenden Ansprüche, wobei der Auffänger (2) $^7$Li umfasst und die nukleare Zusammenwirkung mit dem einfallenden Protonenstrahl $^7$Li(p,n)$^7$Be ist.

12. Vorrichtung nach einem der vorhergehenden Ansprüche, wobei die Ausgangsöffnung (6) für den Neutronenstrahl mindestens einen Abschnitt mit einer der folgenden Geometrien umfasst: zylindrisch, konisch, prismatisch, abgestumpfte Pyramide.

13. Vorrichtung nach einem der vorhergehenden Ansprüche, wobei die Ausgangsöffnung (6) für den Neutronenstrahl eine bewegliche Schließung umfasst, um die Bestrahlung zu stoppen.

14. Vorrichtung nach einem der vorhergehenden Ansprüche, wobei die Abschirmung (7), welche um die Ausgangsöffnung (6) herum für den Neutronenstrahl angeordnet ist, mindestens eins der folgenden Materialien umfasst: LiF, $^6$LiF, B$_4$C, Polyethylen, Pb, Bi.

**15.** Vorrichtung nach einem der vorhergehenden Ansprüche, für deren Verwendung bei der Borneutroneneinfangtherapie.

**Revendications**

**1.** Dispositif de production, de modération et de mise en forme d'un faisceau de neutrons, basé sur la production de neutrons à partir d'un faisceau de protons obtenu par le biais d'un accélérateur de particules, comprenant :

- une ouverture d'entrée (1) à travers laquelle est dirigé le faisceau de protons ;
- une cible (2) disposée dans le trajet de direction du faisceau de protons afin d'interagir de façon nucléaire avec ledit faisceau et produire un faisceau de neutrons qui définit un axe principal de propagation ;
- un module de refroidissement pour refroidir la cible (2) ;
- un modérateur (3) contigu à la cible (2), avec une épaisseur (W2) depuis l'extrémité de la cible (2) et dans la direction du faisceau jusqu'à l'étage de filtration (5), comprise entre 19,62 et 23,98 cm, dans lequel les neutrons produits dans l'interaction nucléaire sont modérés à des énergies du domaine épithermique
dans lequel l'épaisseur totale du modérateur (L4+L5) est comprise entre 24,64 et 36,96 cm, recouvrant la cible (2) et une partie du tube de l'accélérateur de particules vers l'ouverture d'entrée (1),
- un recouvrement réfléchissant (4) entourant le modérateur (3) pour rediriger des neutrons déviés vers l'axe principal et augmenter le flux de neutrons épithermiques ;
- une ouverture de sortie (6) à travers laquelle sort le faisceau de neutrons ; et
- un blindage (7) disposé autour de l'ouverture de sortie (6) pour supprimer les neutrons et le rayonnement gamma qui ne sortent pas du dispositif par ladite ouverture de sortie (6) ; et
- un étage de filtration (5) contigu au modérateur (3) et contigu à l'ouverture de sortie (6) et également entouré par le recouvrement réfléchissant (4), pour filtrer le faisceau de neutrons avant qu'il sorte du dispositif par ladite ouverture de sortie (6) ; dans lequel ledit étage de filtration (5) comprend au moins trois couches : une couche de filtration de neutrons rapide, une couche de filtration de neutrons thermique et une couche de filtration de rayonnement gamma.

**2.** Dispositif de production, de modération et de mise en forme d'un faisceau de neutrons selon l'une quelconque des revendications précédentes, dans lequel la couche de filtration de neutrons rapide comprise dans l'étage de filtration présente une épaisseur (L1) de 1,00 cm $\pm$15%.

**3.** Dispositif de production, de modération et de mise en forme d'un faisceau de neutrons selon l'une quelconque des revendications précédentes, dans lequel la couche de filtration de neutrons thermique comprise dans l'étage de filtration (5) présente une épaisseur (L2) de 0,20 cm $\pm$20%.

**4.** Dispositif de production, de modération et de mise en forme d'un faisceau de neutrons selon l'une quelconque des revendications précédentes, dans lequel la couche de filtration de rayonnement gamma comprise dans l'étage de filtration (5) présente une épaisseur (L3) de 1,00 cm $\pm$15%.

**5.** Dispositif selon l'une quelconque des revendications 2 à 4, **caractérisé en ce que** la couche de filtration de neutrons rapide comprend Al, Fe ou Ni.

**6.** Dispositif selon la revendication précédente, **caractérisé en ce que** la couche de filtration de neutrons rapide est fabriquée avec de l'Al.

**7.** Dispositif selon l'une quelconque des revendications 2 à 6, **caractérisé en ce que** la couche de filtration de neutrons thermique comprend $^{10}$B, $^{6}$Li, Gd, Cd ou LiF, de préférence dans lequel la couche de filtration de neutrons thermique est fabriquée avec du LiF.

**8.** Dispositif de production, de modération et de mise en forme d'un faisceau de neutrons selon l'une quelconque des revendications précédentes, dans lequel le recouvrement réfléchissant (4) entourant le modérateur (3) pour rediriger des neutrons déviés vers l'axe principal, présente une épaisseur (L6) de 25,00 cm $\pm$40% depuis le modérateur jusqu'à l'ouverture d'entrée, dans la direction de the faisceau.

**9.** Dispositif selon l'une quelconque des revendications précédentes, dans lequel le recouvrement réfléchissant (4) comprend au moins l'un des matériaux suivants : Ni, Pb, BeO ou Bi, de préférence dans lequel le recouvrement réfléchissant (4) est fabriqué avec du Pb.

10. Dispositif selon l'une quelconque des revendications précédentes, avec une ouverture de sortie ayant un diamètre A ±2δ, ayant les dimensions radiales suivantes :

diamètre (Ø2) de la partie arrière du modérateur : A±2δ+36 cm ±10%
diamètre (Ø3) du recouvrement (4) : A±2δ+106 cm ±15%
diamètre (Ø4) du dispositif : A±2δ+116 cm ±20%
rayon interne (R1) du collimateur du blindage avant (7) : (0,5A±δ+3 cm ±10%)
rayon externe (R2) du collimateur du blindage avant (7) : (0,5A±δ+22,5 cm ±50%).

11. Dispositif selon l'une quelconque des revendications précédentes, dans lequel la cible (2) comprend du $^7$Li et l'interaction nucléaire avec le faisceau de protons incident est $^7$Li(p,n)$^7$Be.

12. Dispositif selon l'une quelconque des revendications précédentes, dans lequel l'ouverture de sortie (6) pour le faisceau de neutrons comprend au moins une section avec l'une des géométries suivantes : cylindrique, conique, prismatique, pyramide tronquée.

13. Dispositif selon l'une quelconque des revendications précédentes, dans lequel l'ouverture de sortie (6) pour le faisceau de neutrons comprend une fermeture mobile pour arrêter l'irradiation.

14. Dispositif selon l'une quelconque des revendications précédentes, dans lequel le blindage (7) disposé autour de l'ouverture de sortie (6) pour le faisceau de neutrons comprend au moins l'un des matériaux suivants : LiF, $^6$LiF, $B_4C$, polyéthylène, Pb, Bi.

15. Dispositif selon l'une quelconque des revendications précédentes, pour l'utilisation de celui-ci dans la thérapie par capture neutronique au bore.

**FIG. 1**

**FIG. 2**

**FIG. 3**

**FIG. 4**

**FIG. 5**

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1895819 A1

### Non-patent literature cited in the description

- **D. RORER et al.** Current Status of Neutron Capture Therapy, IAEA TECDOC 1223.. *International Atomic Energy Agency, Vienna*, 2011
- **I. PORRAS et al.** Perspectives on Neutron Capture Therapy of Cancer. *CERN Proc.*, 2019, vol. 1, 295-304
- **TORRES-SÁNCHEZ et al.** On the upper limit for the energy of epithermal neutrons for Boron Neutron Capture Therapy. *Radiation Physics and Chemistry*, 2019, vol. 156, 240-244
- **W.S. SNYDER et al.** Estimates of absorbed fractions for monoenergetic photon sources uniformly distributed in various organs of a heterogeneous phantom. *Oak Ridge National Lab, Tenn., J. Nucl. Med.*, 1969, vol. 10 (3), 7-5
- **BRUGGER, R. M. et al.** Rapporteurs' report. Neutron beam design, development, and performance for neutron capture therapy. Springer, 1990, 3-12
- **A. J. KREINER et al.** Present status of accelerator-based BNCT. *Reports of Practical Oncology & Radiotherapy*, 2016, vol. 21 (2), 95-101